Europäisches Patentamt

European Patent Office

Office européen des brevets

(19)

(11) Veröffentlichungsnummer: **0 234 358**
**B1**

(12) # EUROPÄISCHE PATENTSCHRIFT

(45) Veröffentlichungstag der Patentschrift:
30.05.90

(51) Int. Cl.⁵: **A61F 2/36**

(21) Anmeldenummer: **87101572.3**

(22) Anmeldetag: **05.02.87**

(54) **Schaft für eine Hüftgelenksprothese.**

(30) Priorität: **18.02.86 CH 650/86**

(43) Veröffentlichungstag der Anmeldung:
**02.09.87 Patentblatt 87/36**

(45) Bekanntmachung des Hinweises auf die Patenterteilung:
**30.05.90 Patentblatt 90/22**

(84) Benannte Vertragsstaaten:
**AT DE FR GB IT**

(56) Entgegenhaltungen:
**EP-A- 0 128 036**
**DE-A- 3 442 845**
**GB-A- 2 024 631**
**US-A- 4 179 485**
**US-A- 4 536 894**

(73) Patentinhaber: **GEBRÜDER SULZER
AKTIENGESELLSCHAFT, Zürcherstrasse 9,
CH-8401 Winterthur(CH)**

(72) Erfinder: **Griss, Peter, Prof. Dr.-med. Orthopädische
Klinik, im Klinikum der Philipps-Univ. Baldingerstrasse,
D-3550 Marburg(DE)**

(74) Vertreter: **Sparing Röhl Henseler Patentanwälte
European Patent Attorneys, Rethelstrasse 123,
D-4000 Düsseldorf 1(DE)**

## Beschreibung

Die Erfindung betrifft einen sich vom distalen Ende allseitig konisch erweiternden blattartigen Schaft für eine Hüftgelenksprothese, der im proximalen Bereich eine linsenähnliche Scheibenform besitzt und mindestens in diesem Bereich seiner nach anterior bzw. posterior gerichteten Blattseiten mit einer Oberflächenstruktur versehen ist, die in die Linsenform integriert ist.

Verankerungsschäfte der vorstehend genannten Art sind bekannt aus der DE-OS 3 505 997; sie sind für eine zementfreie Verankerung bestimmt, die vorzugsweise im proximalen Bereich des Schaftes erfolgt. Insbesondere aufgrund der Kräfte, die durch die dauernden Be- und Entlastungen infolge des vom Gelenkkopf der Prothese ausgeübten Biegemomentes entstehen, treten bei der bisherigen Konstruktion kleine Schubbewegungen, sogenannte Mikrobewegungen, an der Grenze zwischen dem kortikalen Gewebe des Calcar-Bogens und der mediale Schmalseite des Prothesenschaftes auf.

Derartige Mikrobewegungen sind unerwünscht, da sie ein Anwachsen von Gewebe im Bereich der druckbeanspruchten medialen Schmalseite behindern. Aufgabe der Erfindung ist es somit, das Auftreten derartiger Schubbewegungen zwischen Knochen und Implantat zu verhindern. Diese Aufgabe wird mit der Erfindung dadurch gelöst, dass die Oberflächenstruktur aus einem U-förmig gebogenen Drahtnetz besteht, das die mediale Schmalseite des Schaftes umgreift.

Die Struktur des Drahtnetzes bewirkt, dass eine Verbindung zwischen Knochen und Implantat durch eine Vielzahl von "Poren" im Mikrobereich selbst erfolgt, wobei - besonders wenn das Netz mehrlagig ausgebildet ist - die "innere" Elastizität der einzelnen Lagen des Netzes relativ zueinander mindestens einen Teil der kleinen Schubbewegungen aufnimmt und so von der Grenzfläche Knochen/Netzoberfläche fernhält.

Schaft und Drahtnetz bestehen vorzugsweise aus Titan oder einer Titanlegierung. Es gibt daher vor allem zwei vorteilhafte Möglichkeiten, das Netz auf dem Schaft zu fixieren: Zum einen kann das Netz auf mindestens einen Teil seines Randes mit dem Schaft verschweisst sein und zum anderen erfolgt eine Befestigung mit Hilfe einer mechanischen · Verbindung, beispielsweise durch Nieten.

Im folgenden wird die Erfindung anhand eines Ausführungsbeispiels im Zusammenhang mit der Zeichnung näher erläutert.

Fig. I zeigt den neuen Schaft in einer Aufsicht auf eine seiner Blattseiten in Richtung anterior/posterior;

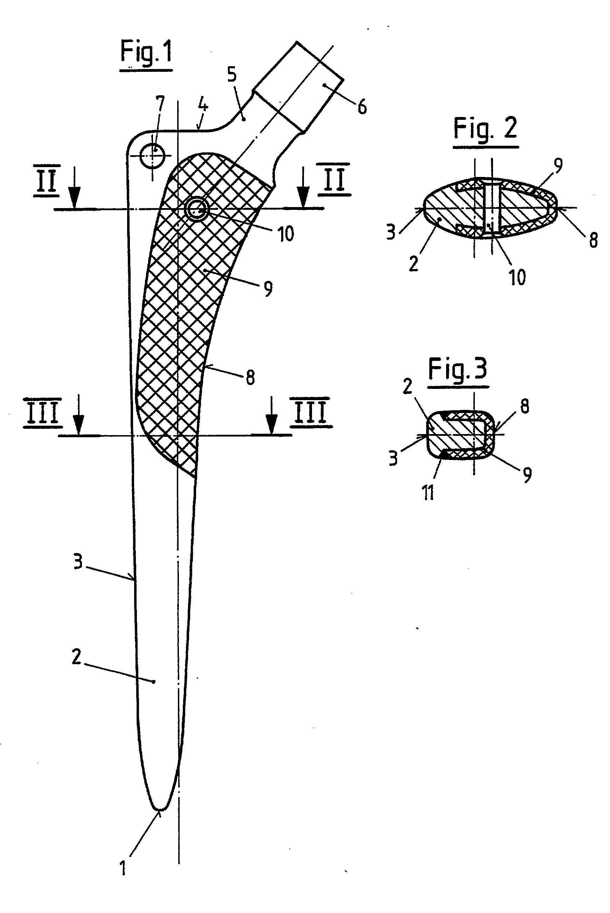

Fig. 2 und 3 sind Schnitte II-II bzw. III-III von Fig. I, wobei in Fig. 2 eine mechanische und in Fig. 3 eine Schweissverbindung zwischen Schaftkörper und Drahtnetz gezeigt sind.

Von seinem distalen Ende I aus erweitert sich der Schaft 2 zunächst konisch nach allen Seiten, wobei seine laterale Schmalseite 3 proximal in eine horizontale Schulter 4 übergeht. Diese setzt sich stufenlos fort in einem Prothesenhals 5, der einen konischen Zapfen 6 zur Aufnahme eines nicht gezeigten Gelenkkopfes trägt. Im Uebergangsbereich von lateraler Schmalseite 3 und horizontaler Schulter 4 ist eine Bohrung 7 für das Eingreifen eines Führungs- und Ausziehinstrumentes vorgesehen.

Die mediale Schmalseite 8 führt in einem Bogen zum medialen Ansatzpunkt des Prothesenhalses 5 an den Schaft 2. Erfindungsgemäss ist als Oberflächenstruktur im proximalen medialen Bereich des Schaftblattes ein Drahtnetz 9 vorgesehen, das bündig in eine entsprechende Vertiefung des Schaftes 2 eingelegt ist und die mediale Schmalseite 8 umfasst. Sein Querschnitt ist im wesentlichen U-förmig; es ist durch mechanische Elemente, z.B. Nieten I0 (Fig. 2) und/oder durch Schweissnähte II (Fig. 3) mit dem Schaft 2 verbunden. Weiterhin kann es aus einer oder mehreren Lagen bestehen, wobei bei mehreren Lagen die Maschenweite oder Porengrösse in den einzelnen Lagen verschieden sein kann, beispielsweise von aussen nach innen abnehmen kann.

## Patentansprüche

I. Sich vom distalen Ende allseitig konisch erweiternder blattartiger Schaft (2) für eine Hüftgelenksprothese, der im proximalen Bereich eine linsenähnliche Scheibenform besitzt und mindestens in diesem Bereich seiner nach anterior bzw. posterior gerichteten Blattseiten mit einer Oberflächenstruktur versehen ist, die bündig mit der linsenförmigen Außenkontur des Schaftes (2) abschließt, dadurch gekennzeichnet, dass die Oberflächenstruktur aus einem U-förmig gebogenen Drahtnetz (9) besteht, das die mediale Schmalseite (8) des Schaftes (2) umgreift.

2. Schaft nach Anspruch I, dadurch gekennzeichnet, dass das Drahtnetz (9) mehrschichtig ausgebildet ist.

3. Schaft nach Anspruch I oder 2, dadurch gekennzeichnet, dass das Netz (9) durch mechanische Verbindungen (I0), beispielsweise Nieten, auf dem Schaft (2) befestigt ist.

4. Schaft nach Anspruch I oder 2, dadurch gekennzeichnet, dass das Netz (9) durch Schweissnähte (II) entlang mindestens einem Teil seiner Ränder auf dem Schaft (2) befestigt ist.

## Claims

1. A blade-like stem (2) for a hip joint prosthesis, the stem widening conically in all directions from its distal end, the blade having in its proximal zone a lens-like discoid shape and having, at least in this zone of its anterior and posterior blade sides, a surface structure terminating flush with the lens-like outer contour of the stem (2), characterised in that the surface structure is in the form of a wire mesh (9) which is bent into the shape of a U and which extends around the medial narrow side (a) of the stem (2).

2. A stem according to claim 1, characterised in that the wire mesh (9) comprises a number of layers.

3. A stem according to claim 1 or 2, characterised in that the mesh (9) is secured by mechanical connections (10), for example, rivets, to the stem (2).

4. A stem according to claim 1 or 2, characterised in that the mesh (9) is secured to the stem (12) by weld seams (11) along at least some of its edges.

**Revendications**

1. Tige (2) du type spatule pour une prothèse coxofémorale, qui présente un élargissement conique de toutes parts à partir de l'extrémité distale, possède une configuration discoïdale du type lentiforme dans la région proximale et est pourvue, au moins dans cette région de ses faces respectivement tournées vers la zone antérieure ou postérieure, d'une structure superficielle qui vient à fleur du pourtour extérieur lentiforme de la tige (2), caractérisée par le fait que la structure superficielle consiste en un entrelacs (9) de fils métalliques cintré en U, qui entoure le petit côté médial (8) de la tige (2).

2. Tige selon la revendication 1, caractérisée par le fait que l'entrelacs (9) de fils métalliques est réalisé en plusieurs couches.

3. Tige selon la revendication 1 ou 2, caractérisée par le fait que l'entrelacs (9) est fixé sur la tige (2) par des solidarisations mécaniques (10), par exemple des rivets.

4. Tige selon la revendication 1 ou 2, caractérisée par le fait que l'entrelacs (9) est fixé sur la tige (2) par des cordons de soudure (11), le long d'au moins une partie de ses bords.

Fig.1

Fig. 2

Fig.3